(19) Europäisches Patentamt
European Patent Office
Office européen des brevets

(11) EP 1 950 217 A1

(12) EUROPEAN PATENT APPLICATION

(43) Date of publication:
30.07.2008 Bulletin 2008/31

(21) Application number: 07101258.7

(22) Date of filing: 26.01.2007

(51) Int Cl.:
*C07F 17/02* (2006.01) *C07F 15/00* (2006.01)
*A61K 31/28* (2006.01) *A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Université de Neuchâtel**
**2001 Neuchâtel 1 (CH)**

(72) Inventors:
- **Therrien, Bruno**
  **2000, Neuchâtel (CH)**
- **Padavattan, Govindasvamy**
  **2000, Neuchatel (CH)**
- **Süss-Fink, Georg**
  **2000, Neuchâtel (CH)**
- **Ang, Wee Han**
  **Cambridge, MA 02140 (US)**
- **Dyson, Paul Joseph**
  **1024, Ecublens (CH)**
- **Juillerat, Lucienne**
  **1000, Lausanne 26 (CH)**

(74) Representative: **Schneiter, Sorin et al**
**Abrema Agence Brevets & Marques**
**Avenue du Théâtre 16**
**P.O. Box 5027**
**CH-1002 Lausanne (CH)**

# (54) Organometallic compounds for the treatment of cancer

(57) The present invention relates to novel organometallic compounds for use as a medicine, in particular in photodynamic therapy against various diseases, such as cancer. The compounds comprise a central porphyrin or phtalocyanine backbone to which ligand linkers coordinated to at least one transition metal are attached. Eta-5 or eta-6 arenes further bind to the transition metal. According to a preferred embodiment, the compound is a tetranuclear Ruthenium (+II) complex.



Printed by Jouve, 75001 PARIS (FR)

## Description

### Technical Field

The present invention relates to organometallic compounds, uses of the compounds in the treatment of cancer, in photodynamic therapy, as pharmaceutically active principles, and to a method of treating cancer.

### Background of the Invention and Problem to Be Solved

Cisplatin, usually in combination with other drugs, is still the treatment of choice for more than 50% of all cancer patients. It shows good activity against testicular, ovarian, oropharyngeal, bronchogenic, cervical and bladder carcinomas, lymphoma, osteosarcoma, melanoma and neuroblastoma. However, it exhibits high toxicity and in general the treatment generates undesirable side-effects, such as nephrotoxicity and gastrointestinal toxicity. Moreover, it is inactive against many cancer cell lines and also against metastasis cancers. Therefore, the development of new drugs, such as metal-based ones, with reduced side-effects, and with equivalent or better activity than cisplatin has been the focus of much research, and is also an objective of the present invention.

In photodynamic therapy (PDT) of cancer, porphyrin derivatives are used as photosensitising agents. By exploiting photochemical and photobiological processes initiated by the porphyrin core, irreversible damages are created to tumor cells. In US 7,087,214 platinum(II)-porphyrin complexes are disclosed, in which chemotherapeutic and photodynamic properties are combined. Of course, the above-indicated disadvantages are also observed in the complexes of this reference.

Many other compounds have been suggested in different types of therapies against different types of cancers. For example, Jang-Qiang Wang et al. "Porphyrin-carborane organometallic assemblies based on 1,2-dicarba-closo-dodecacarborane (12) ligands", Chem. Comm., 2006, 162-164, propose rhodium and zinc containing complexes in Boron Neutron Capture Therapy. In WO 02/40494, a completely different approach is disclosed, based on ruthenium-aryl-compounds comprising a 1,3,5-triaza-7-phosphadamatane ligand, which are used in cancer therapy. So far, however, compounds resulting from these recent efforts have not yet reached the stage of marketed products.

In view of the prior art, it is an objective of the present invention to provide other, different compounds that can be used in the treatment of cancer, for example in photodynamic therapy. Preferably, these compounds have a reduced general toxicity if compared to cisplatin. It is desired that these compounds exhibit less side-effects than cisplatin and are also effective against primary cancers and metastases.

### Summary of the Invention

In a first aspect, the present invention provides an organometallic compound comprising a structure of formula (I):

$$[M_n\ Mc\ (A)_n\ (L)\ (B)_p\ (C)_q] \qquad (I)$$

which is charged or neutral and which may be present in the form of a salt and/or an optically resolved enantiomer, and, wherein:

M is a transition metal selected from Ru, Rh, Os, Ir, and Fe;

n is an integer of 1 to 12 if L corresponds to formula (II) below and an integer of 1 to 16 if L corresponds to formula (III) below and defines the number of metal atoms M present in compound (I);

Mc is an optional metal in the centre of L, which may be selected from the same metals as M, and from metals including Zn, Ni, Co, Cu, Ag, Au, Cd, Hg, Pd, and Pt.

A and L are ligands of M;

B and C are, independently of each other, optional ligands of M, with p and q, independently of each other, being 0 or an integer of 1 to n.

wherein, if n > 1, all n ligands A and all n metals M in the compound of formula (I) can be the same or different; and, accordingly, wherein, if p and/or q > 1, all p ligands B and/or all q ligands B, respectively, in the compound of formula

(I) can be the same or different;

A is an $\eta^6$- arene or a $\eta^5$-cyclopentadienyl, or a $\eta^5$-cyclopentadienyl derivative;

B and C are, independently of each other, freely selectable ligands capable of binding to M, wherein:

B and C may be covalently connected with each other so as to form a single, bidentate chelating ligand (B-C), or, alternatively,
any one or both of B and C, may be covalently bound to A, so as to form a multidentate chelating ligand (A-C), (A-B) or (A-B-C);

L is a porphyrin derivative of formula (II) or a phtalocyanine derivative of formula (III) below:

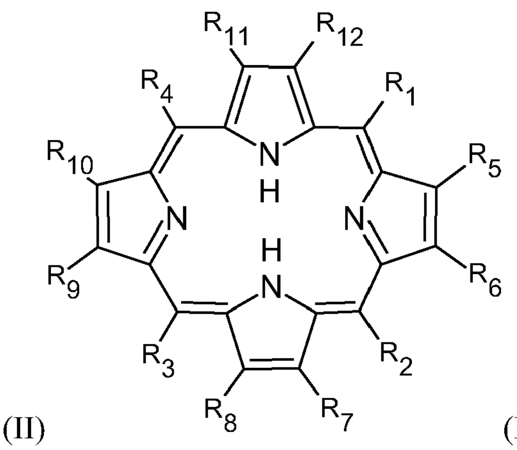

(II)

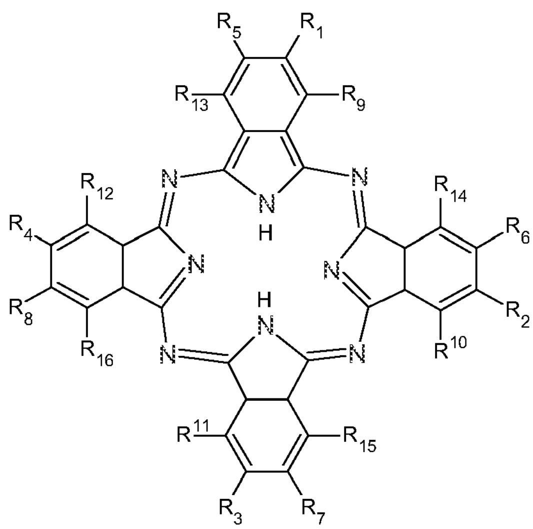

(III)

wherein:

m residues of $R_1$ - $R_{12}$ if L = (II) or of $R_1$ - $R_{16}$ if L = (III) are linkers comprising, all m residues together, at least n donor groups for M, wherein each of said at least n donor groups is selected independently from S-, O-, N- and/or P-donor groups, and,
m is an integer of 1 to 12 if L = (II) and an integer of 1 to 16 if L = (III),
wherein:
two or more (r) of said m residues of $R_1$ - $R_{12}$ if L = (II) or of $R_1$ - $R_{16}$ ifL = (III) may, independently of the remaining (m - r) residues, be covalently connected with each other to form a system comprising at least one ring fused to the porphyrin structure of formula (II) or to the phtalocyanine structure of formula (III), respectively; and, wherein:
(12-m) residues of $R_1$ - $R_{12}$ if L = (II) or (16-m) of $R_1$ - $R_{16}$ if L = (III), respectively, are selected, independently of each other, from H, C1-C20 alkyls, C2-20 alkenyls, C2-C20 alkynyl, C6-C22 aryls, said alkyls, alkenyls, alkynyls and aryls being optionally substituted and optionally comprising one or more heteroatoms, said optionally substituted alkyls, alkenyls, alkynyls being linear or cyclic.

In a second aspect, the present invention provides the use of the compounds of the present invention as pharmaceutically active principles, in particular as medicaments.
In a third aspect, the present invention provides a method of treatment of cancer, the method comprising the step of administering to an individual a pharmaceutically active quantity of the compound of the present invention.
The compounds according to the present invention were found to have excellent properties in photodynamic therapy.

## Brief Description of the Drawings

**Figure 1** shows UV-visible absorption spectra of TPP (5,10,15,20-tetra(4-pyridyl)porphyrin) and compounds of the present invention in dichloromethane at 298 K.

**Figure 2** is a ORTEP representation of $[Ru_4(\eta^6\text{-}C_6Me_6)_4(TPP)Cl_8]$ (4), at 50% probability level, and with hydrogen atoms being omitted for clarity.

**Figure 3** is a ORTEP representation of $[Rh_4(\eta^5\text{-}C_5Me_5)_4(TPP)Cl_8]$ (7), at 50% probability level, and with hydrogen atoms being omitted for clarity.

**Figure 4** shows response curves of two melanoma cell lines (ME300 and ME275) after 72 h of exposure to various concentrations of a compound ($[Ru_4(\eta^6\text{-}C_6H_6)_4(TPP)Cl_8]$) of the present invention.

**Figure 5** shows response curves of two melanoma cell lines (ME300 and ME275) after 72 h of exposure to various concentrations of a compound ($[Ru_4(\eta^6\text{-}C_6Me_6)_4(TPP)Cl_8]$) of the present invention.

## Detailed Description of the Preferred Embodiments

The compounds of the present invention comprise a structure of formula (I), $[M_n\ Mc\ (A)_n\ (L)\ (B)_p\ (C)_q]$, wherein M is selected from Ru, Rh, Os, Ir, and Fe, including combinations of two or more of these. For example, if n is 4, $M_1$ and $M_3$ may be Ru, with $M_2$ and $M_3$ being Os in the compound of the invention. Preferably, M is selected from the group consisting of Ru, Rh, Os, Ir, and combinations including two or more of these. More preferably, M is selected from the group of Ru and Os and combinations thereof. While different metals M may be combined if n >1, the compound of the present invention preferably comprises only one transition metal M. Most preferably M is Ru. The transition metal M may be present at any oxidation state, for example +IV, +III, +II. Different oxidation states may be present within the same molecule. Preferably, M is at the oxidation state +II.

Preferably, the optional metal Mc is absent, the compound of formula (I) of the present invention corresponding to formula $[M_n\ (A)_n\ (L)\ (B)_p\ (C)_q]$. In other words, the compounds of the present invention are preferably free of a central metal Mc. However, it is easy to add a central metal to the compounds of the present invention, for example for the purpose of modifying the charge of the overall compound or for other reasons, including non-technical ones. Therefore, the present invention also encompasses compounds in which a central metal atom is present. The central metal Mc, if present, is coordinated to the centre of L, which is described in further detail below.

The letter n designates the number of metals M in the compound of the present invention, and is therefore an integer. Theoretically, n is only limited by the possibility of adding suitable ligand linkers to the central structure L (see below) and could therefore be in the ranges of 10-20, 3-30, 15-50 or even higher. However, simpler specimen of the present invention will generally comprise less metal atoms, n thus being in the range of 1-16 or 1-12. Preferably, n is an integer of 1-10, more preferably 2-8, even more preferably 3-6. Most preferably, n is 4.

L is a compound according to the structure of formula (II) or (III) shown above, wherein at least one of the residues $R_1$-$R_{12}$ in case of structure (II) and $R_1$-$R_{16}$ in case of structure (III) comprises at least one donor group, which binds, by a typical ligand bond, to the metal M.

For the purpose of the present invention, the reference to a group of consecutively numerated residues $R_X$-$R_Y$, for example in the phrase "at least one of the residues $R_{1\text{-}16}$" or "any of $R_{1\text{-}16}$" is understood as a reference to all residues included in the range. For example, "at least one of residues $R_1$-$R_{16}$" means at least one of the group of $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$". "Two of $R_1$-$R_{16}$" means two different residues picked randomly from this group, and so forth.

With respect to L, the expression "a residue R" is to be understood as "one of the residues $R_1$-$R_{12}$ if L = (II) and one of $R_1$-$R_{16}$ if L = (III)". The expression "any R" means "any R of $R_1$-$R_{12}$ if L = (II) and at least one of $R_1$-$R_{16}$ if L = (III)", and so forth.

L may have one or more residues, namely m residues, carrying, altogether, at least n donor groups so that L functions as a ligand to all n metals M. In other words, any residue R, for example $R_1$, may carry one or more donor groups and thus form a ligand to an according number of metals M. It is also possible that any R comprises one or more chelating groups, so that two or more donor groups of the respective residue bind to the metal M, similar to the situation shown in US 2004/0023942, Figure 1. Furthermore, one can envisage two residues R forming a chelating ligand for one M, similar to the situation in US 2004/0023942, Figure 2. This document is entirely incorporated herein by reference.

As the skilled person will understand, at least one of the residues R of L, but potentially all of them, is a linker

that comprises a donor group so as to bind to metal M. This linker is also referred to herein as "ligand linker" or "linker residue". Now, the present invention does not intend to impose any limitation as to the structure of this linker. To date, a great number of such linkers is available and the mentioning of all of them would go beyond the information that can be listed in this document. However, for the purpose of providing a guideline, further indications concerning the possible structure of the ligand linker are given below. It is recalled, however, that there are nearly no limits with respect to the concrete structure that is used, and which one(s) of the residues R of L forms a ligand linker.

Accordingly, any such linker residue R of L is preferably a C1-C22, more preferably C2-C15 and most preferably C3-C10, linear, branched and/or cyclic hydrocarbon comprising an S-, O-, N-, and/or P-donor group. Of course, the linker residue may be further substituted and may comprise one or more heteroatoms.

S-donor groups are groups which bind to a metal M via a sulphur atom. They are well known in the art and include: sulfoxide groups ($R^{S1}R^{S2}SO$), thioether groups ($R^{S1}R^{S2}S$), thiolate groups ($R^{S1}S^-$); sulfinate groups ($R^{S1}R^{S2}SO$); and sulfenate groups ($R^{S1}SO^-$),

wherein $R^{S1}$ and $R^{S2}$ are independently selected from C1-C20 alkyl, C2-C20 alkenyl, C2-C20 alkynyl and C5-C22 aryl, which may be linear, cyclic and/or branched, which are optionally substituted and which optionally comprises one or more heteroatoms, and where at least one of $R^{S1}$ and $R^{S2}$ is a bivalent substituent bound to L, thus forming a corresponding residue R. Of course, it is possible that if two residues $R^{S1}$ and $R^{S2}$ if both present are present, are formed from two residues R of L.

Residues comprising O-donor groups are residues which bind to a metal M via an oxygen atom. They are well known in the art and include: carboxylate ligands ($R^CCO_2^-$); and sulfonate ligands ($R^{S1}SO_3^-$), wherein $R^C$ and $R^{S1}$ are defined as $R^{S1}$ and $R^{S2}$ above.

Residues comprising N-donor groups are residues which bind to a metal M via an nitrogen atom. They are well known in the art and include nitrile ligands (N≡C-RN); azo ligands (N=N-RN), aromatic N-donor ligands, and amine ligands ($NR^{N1}R^{N2}R^{N3}$), for example.

In both nitrile and azo ligands R is defined as $R^{N1}$ and $R^{N2}$ above.

Aromatic N-donor ligands include optionally substituted pyridine, pyradizine, pyrimidine, pyrazine, imidazole, purine, 1,8-naphthydrin, chinoxaline, chinazoline, pteridine, to mention a few examples.

With the amine ligands, $R^{N1}$, $R^{N2}$ and $R^{N3}$ may be independently selected from H and C1-C10 alkyl, C2-C20 alkenyl, C2-C20 alkynyl and C5-C22 aryl, they may be linear, cyclic and/or branched, and optionally be substituted and optionally comprising one or more heteroatoms, with the proviso that at least one of $R^{N1,}$ $R^{N2}$ and $R^{N3}$ is a bivalent substituent bound to L, thus forming a corresponding residue R. It is, of course, possible that two or all three of $R^{N1}$ - $R^{N3}$ are connected to each other to form a cyclic residue comprising an N-donor group.

According to an embodiment of the present invention, each of said linkers comprises, independently of the others, at least one aromatic N-ring or at least one amine functioning as said N-donor group.

Residues comprising P-donor groups are residues which bind to a metal M via an phosphorous atom. They are well known in the art and include phosphine ligands ($R^{P1}R^{P2}R^{P3}P$) and phosphite ligands $P(OR)_3$, wherein the 3 residues (OR) of the phosphite ligand may be different, resulting in a formula ($P(OR^{P1})(OR^{P2})(OR^{P3})$), wherein $R^{P1}$, $R^{P2}$, and $R^{P3}$ in said phosphine or phosphite ligand are, independently of each other selected from C1-C20 alkyl, C2-C20 alkenyl, C2-C10 alkynyl and C5-C22 aryl, they may be linear, cyclic and/or branched, and optionally are substituted and optionally comprise one or more heteroatoms, with the proviso that at least one of $R^{N1}$, $R^{N2}$ and $R^{N3}$ is a bivalent substituent bound to L, thus forming a corresponding residue R. It is, of course, possible that two or all three of $R^{N1}$ - $R^{N3}$ are connected to each other to form a cyclic residue comprising an P-donor group. An example of such a compound is PTA (1,3,5-triaza-7-phosphaadamantane).

A particularly preferred linker ligand R of L comprising an N-donor group is the ligand of formula (VI).

(VI) 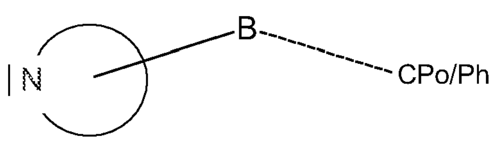

wherein:

- the circle represents a mono- or polycyclic system, optionally substituted, comprising at least one N-heteroatom, indicated as N, which provides an electron pair, indicated as vertical line, enabling attachment to M;
- B is an optional connecting unit, which may be selected from alkylene, alkenylene, alkynylene and arylene, which is optionally substituted, which optionally comprises one or more heteroatoms and has 0-15, preferably 1-8 carbon atoms;

The dotted line represents a bond to a carbon of the porphyrin or phtalocyanine of formula (II) or (III), indicated as CPo/Ph, which is itself not part of the linker, but a carbon carrying any residue R of L.

Preferably, in formula (VI), the circle is a heterocyclic ring, for example a heterocyclic arene.
Preferably, it is a 5- or 6-membered heterocyclic ring, for example arene. Preferably, B is a $C_1$-$Cl_6$, more preferably a $C_2$-$C_4$ alkylene.
According to a particularly preferred embodiment, any one of the ligands R of L is a N-donor ligand comprising the structure of formula (VII) below:

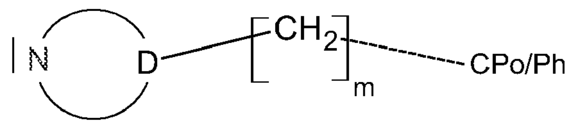

(VII)

the circle represents a mono- or polycyclic system, which is optionally substituted, comprising at least one N-heteroatom, indicated as N, which provides an electron pair, indicated as vertical line, enabling attachment to M;
D is a carbon or nitrogen atom;
The dotted line represents a bond to a carbon of the porphyrin or phtalocyanine of formula (II) or (III), indicated as CPo/Ph, which is itself not part of the linker, but a carbon carrying any residue R of L.
m is 0-10, preferably 1-6, most preferably 3-4.
The cyclic system may, for example, be selected from pyridine, imidazole, purine, pyrazine, pyrimidine, 1,8-naphthydrin, chinoxaline, chinazoline, pteridine. Preferably, the cyclic system is imidazole, resulting in N-donor ligands comprising structures as illustrated in formula (VIII) and (IX) below.

(VIII) 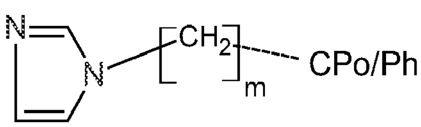 (IX) 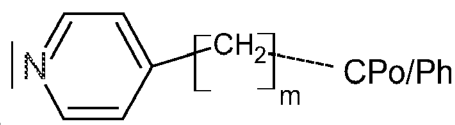

wherein m, the dashed line and CPo/Ph have the same meanings as for formula (VII) above.
According to another, preferred embodiment, one or more R of L is a linker ligand of formula (X), more preferably (XI) below.

(X) 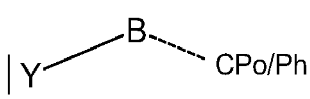 (XI) 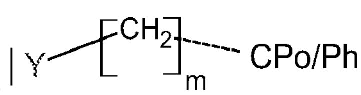

wherein Y can be a N-, O-, S- or P-donor group such as defined above, for example $-NH_2$ or $-COO^-$; B, m, the dashed line, and CPo/Ph have the same meanings as for formula (VI) or (VII) above.
Two or more residues R of L may bind to the same metal M in compound (I), acting as a bidentate chelating ligand. For example, two ligands may have a structure of formula formulae (XII) or (XIII) below.

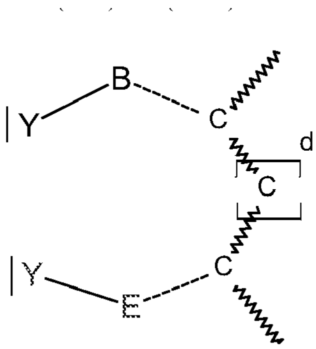

(XII)

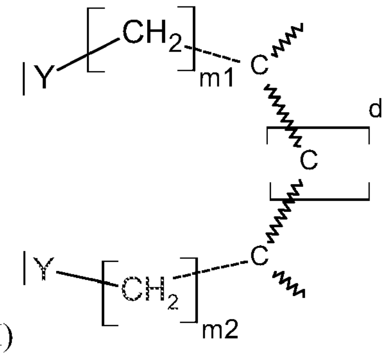

(XIII)

wherein Y is as in (X) above; B and the dashed line are as in formula (VI) above; E is selected from alkylene, alkenylene, alkynylene and arylene, which is optionally substituted, which optionally comprises one or more heteroatoms and has 1-15, preferably 2-8 carbon atoms, wherein B and E may be the same or different; C denotes carbons of the porphyrin or phtalocyanine backbone of formulae (II) or (III) to which the linker ligand is covalently bound (dashed line), and the curved lines design bonds between carbons of said (II) or (III) backbone, which may be single or double bonds, but preferably comprise conjugated double bonds. Small m1 and m2 may are integers of 1-10, preferably 2-5, and may be the same or different. As is indicated by d, which is 0 or an integer of 1- 10, preferably 1-3, the residues carrying the donor group may be attached to neighbouring carbons C or may be separated by up to d carbons of the porphyrin or phtalocyanine backbone (II) or (III), respectively.

For providing another example, any residue R of L as shown in (II) or (III) can also be selected from residues having the structure of formula (XIV):

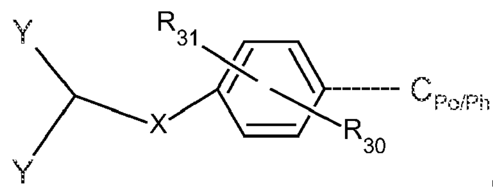

(XIV)

wherein the dotted line and $C_{Po/Ph}$ are defined as in formula (VI) above; Y is defined as in formula (X) above; $R_{30}$ and $R_{31}$ may independently of each other be selected from H, alkyls and alkenyls, for example; X may be selected from $-[CH_2]_m-$ as defined in formula (VII) above, -O-, -S-, -NH-, and from -N(-alkyl)-. Preferably, Y is -COO- or $-NH_2$.

As the examples above illustrate, the constitution of the linker ligands forming at least one of residues $R_1$-$R_{12}$ and $R_1$-$R_{16}$, respectively, is not restricted to a specific structure, but may be chosen by the skilled person's preferences.

The compounds of the present invention comprise an arene or cyclopentadienyl, or a derivative thereof, which is preferably bound by a $\eta$6- or $\eta$5-, respectively, bond to M. Preferably, the arene and/or the cyclopentadienyl derivative is a C6-C30, more preferably C6-C22, even more preferably C6- C12 arene or cyclopentadienyl derivative, optionally comprising one or more heteroatoms.

For example, A may be a mono-, oligo-or polycyclic system, comprising two or more rings fused together. Examples are of A thus include benzene, naphthalene, anthracene, indene, biphenylene, which are optionally substituted and which optionally comprise one or more heteroatoms.

According to a preferred embodiment of the organometallic compound of the present invention, is a compound of formula (IV) or (V):

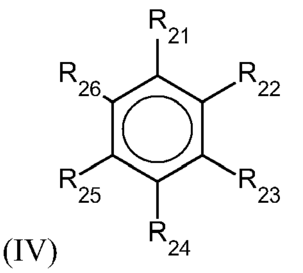
(IV)

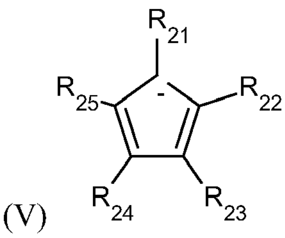
(V)

wherein, if A is (IV), any one of $R_{21}$-$R_{26}$, or, if A is (V), respectively, any one of $R_{21}$-$R_{25}$, is independently selected from H, C1-C20 alkyl, C2-20 alkenyl, C2-C20 alkynyl, C6-C22 aryl, said alkyl, alkenyl, alkynyl being linear or cyclic, and said alkyl, alkenyl, alkynyl and aryls being optionally substituted and optionally comprising one or more heteroatoms, wherein any of the residues $R_{21}$-$R_{26}$ or $R_{21}$-$R_{25}$, respectively may be covalently connected with one or more other residues of $R_{21}$-$R_{26}$ or $R_{21}$-$R_{25}$, respectively, to form one or several rings fused to the ring of formula (IV) or (V), respectively.

Preferably, if A is (IV) any one of $R_{21}$-$R_{26}$, or, if A is (V), respectively, any one of $R_{21}$-$R_{26}$, is selected, independently from each other, from H, C1-10-alkyl, C1-10-alkoxy, C1-10 ether, and C1-10 ester.

For example, A is benzene, 1,2-dimethylbenzene, 1,3-dimethylbenzene, 1,4-dimethylbenzene, 1,3,5-trimethylbenzene, 1,2,4-trimethylbenzene, durene, methyl-benzene, p-cymene, hexamethylbenzene, cyclopenta-1,3-diene, 1,2,3,4,5-pentamethylcyclopenta-1,3-diene, diethylterephtalate.

B and C are, as indicated above with respect to the structure of formula (I), $[M_n\ Mc\ (A)_n\ (L)\ (B)_p\ (C)_q]$, independently of each other, optional ligands of M, with p and q, independently of each other, being 0 or an integer of 1 - n.

B and C are uncommitted ligands capable of binding to M, which means that they may be freely selected. For example, they may be provided by solvent molecules. According to a preferred embodiment, A and B are, independently of each other, selected from H, F, Cl, Br, I, OH, OMe, OEt, OBu, $OH_2$, $CH_3CN$, $(CH_3)_2CO$, DMSO, THF, CO, $CF_3SO_3$, $C_2O_4$, phosphine ligands as generally defined above with respect to ligands comprising an P-donor group, for example $PMe_3$, $PEt_3$, $PBu_3$, $PPh_3$, $PCy_3$, acetate, acetylacetonate, pyridine, pyrimidine, pyrazine, imidazole, pyridazine, phthlalazine, quinoxaline, 1,5-naphthyridine, 1,8-naphthyridine, 2,2'-bipyrimidine, phenazine, azobenzene, 2,2'-azopyridine, 2,3-di-(2'-pyridino)quinoxaline, diphenylphosphineethane, pyrazinecarboxylic acid, pyrazinedicarboxylic acid, DABCO, ethylenediamine, 1,3,5-triaza-7-phosphatricyclo[3.3.1.1$^{3,7}$]decane (PTA).

More preferably, B and C are, independently of each other, selected from $H_2O$, Cl, Br, I, CO, SH, OH, Pyridine, $PPh_3$, PTA.

The compound (I) of the present invention may comprise p ligands B and q ligands C. All q ligands B of a compound (I) may be the same or different, and all p ligands C of said compound (I) may be the same or different. Preferably, all p ligands B are identical. Similarly, all q ligands C are preferably identical.

Small p and q may be different, but preferably p = q. Even more preferably, n = p = q, which means that for every metal M, one B and one C is present, binding to that metal.

B and C may be covalently connected with each other so as to form a single, bidentate chelating ligand (B-C). Examples of such chelating ligands are ethane-1,2-diamine, propane-1,2-diamine, benzene-1,2-diamine, cyclohexane-1,2-diamine, malonate, 2,2-bipyridine, pyrazinecarboxylic acid, oxalate, quadratic acid, quinoxaline, and 2,2-bipyrimidine.

Alternatively, B and C, may be covalently bound to A, so as to form a multidentate chelating ligand (A-B), (A-C), or (A-B-C). (A-C) and (A-B) include compounds of formula (IV) and (V), in which one of the residues $R_{21\text{-}26}$ and $R_{21\text{-}25}$, respectively, comprises an S-, O-, N- and/or P-donor group as defined above. To provide some arbitrary examples of ligands (A-B) or (A-C) one could mention 2-phenylethanamine, 2,2-bipyridine, 2,2-bipyrimidine.

(A-B-C) include compounds of formula (IV) and (V), in which two of the residues $R_{21}$-$_{26}$ and $R_{21}$-$_{25}$, respectively, comprises an S-, O-, N- and/or P-donor group as defined above. An example for such a compounds would be 2,2'-(1,4-phenylene)diethanamine, 2,2-bipyridine, 2,2-bipyrimidine.

According to preferred embodiment of the present invention B and C are separate compounds individually bound to M. Preferably, B and C are the same compound.

In the present specification reference to "substituents" is repeatedly made, for example in the expression "optionally being substituted", in particular with respect to residues, structures or compounds in particular residues $R_1$-$R_{12}$ of the structure of formula (II), residues $R_1$-$R_{16}$ of the structure of formula (III), residues $R_{21}$-$R_{26}$ of (IV), $R_{21}$-$R_{25}$ of (V), residues of ligand linkers comprising S-, O-, N-, and/or P-donor groups, namely of $R^{S1}$ > $R^{S2}$ ,$R^C$ ,$R^{N1}$, $R^{N2}$ , $R^{N3}$ ,$R^{P1}$ , $R^{P2}$ ,$R^{P3}$, with respect to ligand linkers (VI), (VII), (VIII), (IX), (X), (XI), (XII), (XIII) and (XIV).

These substituents may be selected, independently from each other within each residue, from linear, branched and cyclic C1-C20 alkyls, alkenyls, and/or alkynyls; halo (-Cl, -Br, -F, -I); keto (=O); hydroxy (-OH); carboxy (-COOH); amino (e.g. $-NH_2$); cyano (-C≡N); nitro ($-NO_2$); ( mercapto (-SH); thiooxo (=S); sulfo ($-SO_3H$); sulfino ($-SO_2H$); sulfeno

(-SOH). Substituents may comprise heteroatoms and may be further substituted as defined herein.

Heteroatoms, for the purpose of the present specification, include all heteroatoms, but in particular B, Al, Si, O, S, Se, N, P, As, halo, Li, Na, K, Be, Mg, Ca, Sr, for example. Preferred heteroatoms are O, S, N, P, and halo.

Examples of C1-C10 alkyls include saturated alkyls, such as methyl (C1), ethyl (C2), propyl (C3), butyl (C4), pentyl (C5), hexyl (C6), heptyl (C7), octyl (C8), nonyl (C9), decyl (C10); saturated linear alkyls such as methyl (C1), ethyl (C2), n-propyl (C3), n-butyl (C4), n-pentyl (C5), n-hexyl (C6), n-heptyl (C7), n-octyl (C8), n-nonyl (C9), n-decyl (C10), cyclic alkyls such as cyclopropyl propyl (C3), cyclobutyl (C4), cyclopentyl (C5), cyclohexyl (C6), cycloheptyl (C7), cyclooctyl (C8), cyclononyl (C9), cyclodecyl (C10); branched alkyls such as iso-propyl (C3), iso-butyl (C4), sec-butyl (C4), tert-butyl (C4), iso-pentyl (C5) and neo-pentyl (C5).

C2-C10 alkenyls include ethenyl (vinyl, -CH=CH2), 1-propenyl ($-CH=CH-CH_3$), 2-propenyl (allyl, $-CH_2-CH=CH_2$), isopropenyl (1-methylvinyl, $-C(CH_3)=CH_2$), butenyl (C4), pentenyl (C5), hexenyl (C6) and so forth; cyclic alkenyls include cyclopropenyl, cyclobutenyl (C4), cyclopentenyl (C5), cyclohexenyl (C6), cycloheptenyl (C7), cyclooctenyl (C8), cyclononenyl (C9), cyclodecenyl (C10).

According to an embodiment of the present invention, in the compound comprising a structure of formula (I), n is an integer of 1 to 4 and m = n.

According to another embodiment n is an integer from 1 to 4, m = n, L is (II), all n residues are selected from R1-R4, and, each of the n linkers comprises at least one S-, O-, N- or P-donor group.

Preferably, M is $Ru^{II}$, n = 4, R1 to R4 are all identical and each is a linker comprising one S-, O-, N-, or P- donor group.

More specifically, the compound of formula (I) is $[Ru_4 (A)_4 (L) (B)_4 (C)_4]$, with A, L, B, and C being defined as above.

According to a preferred embodiment of the present invention, the compound of the present invention is $[Ru_4 (A)_4 (TPP) (B)_4 (C)_4]$, wherein TPP is 5,10,15,20-tetra(4-pyridyl)porphyrin and A, B, C are defined as above. More preferably, the compound of the invention is $[Ru_4 (\eta^6\text{-arene})_4 (TPP) Cl_8]$. For example, $\eta^6$-arenes are selected from benzene ($C_6H_6$), methyl-benzene ($C_6H_5CH_3$), 1-isopropyl-4-methylbenzene ($p$-$Pr^iC_6H_4Me$), hexamethylbenzene ($C_6Me_6$) and diethyl terephtalate.

The compounds of the present invention may be used as pharmaceutically active principles. For example, they are useful as medicaments. The compounds of the present invention are preferably used as medicaments against cancer. Accordingly, the present invention relates to the use of the organometallic compounds comprising a structure of formula (I) in therapeutic cancer treatment.

The invention provides compounds comprising a structure of formula (I), or prodrugs or solvates thereof ("active compounds"), for use in a method of treatment of the human or animal body. A method of treatment may comprise administering to such an individual a therapeutically-effective amount of the compound of the present invention, preferably in the form of a pharmaceutical composition. The term treatment as used herein in the context of treating a condition, pertains generally to treatment and therapy, whether of a human or animal (e.g. in veterinary applications), in which some therapeutic effect is achieved, for example, the inhibition of the progress of the condition, and includes a reduction in the rate of the progress, a halt in the rate of the progress, amelioration of the condition, and cure of the condition. The condition usually is associated with suffering, from psychological and/or physical pain, with the individual being in need of a treatment. Treatment as a prophylactic measure (i.e. prophylaxis) is also included.

The compound of the invention or pharmaceutical composition comprising the active compound may be administered to an individual by any convenient route of administration, whether systemically /peripherically or at the site of desired action, including but not limited to, oral (e.g. by ingestion), topical (including e.g. transdermal, intranasal, ocular, buccal and sublingual), pulmonary (e.g. by inhalation or insufflation therapy using an aerosol, e.g. through mouth or nose), rectal, vaginal, parenteral, for example by injection, including subcutaneous, intradermal, intramuscular, intravenous, intraarterial, intracardiac, intrathecal, subcuticula, subcapsular, intraorbital, intraperitoneal, intratracheal, subarachnoid, and intrasternal, by implant of a depot (e.g. subcutaneously or intramuscularly).

The compound of the present invention is particularly useful in photodynamic therapy (PDT).

With respect to the method of treatment of cancer according to the present invention, said method preferably comprises the further step of exposing said subject to light of a specific wavelength and/or intensity. The optimal wavelength will depend on various factors, such as the specific disease treated, the porphyrin or phtalocyanine core, and, in case of cancer treatment, the position of the cancer in the human body, for example. The skilled person will easily determine the optimal value. As a starting point, one can use wavelength of 400-800 nm, with a 200 mW halogen light source equivalent to a light exposure dose of 1.2 $J/cm^2$.

The compound of the present invention may be administered alone, but is preferably presented as a pharmaceutical composition (e.g. formulation) comprising at least one active compound together with at least one or more pharmaceutically carriers, buffers, as mentioned in WO 2006/018649, p. 16-20, "formulations", which reference is expressly incorporated herein by reference.

Similar to the wavelength and light intensity, the optimal compound concentration will need to be determined,

by the skilled person, in dependency of the disease treated, the treatment regime, and so forth. So far, plasma concentrations in the ranges of 0.5 to 1000μM, preferably 1 to 500 μM and most preferably 5 to 100 μM are considered to be efficient.

The present invention is described more concretely with reference to the following examples, which, however, are not intended to be understood as any kind of restriction of the scope of the present invention.

## Examples

Examples 1 - 10: Synthesis and Characterisation of Compounds of the Present Invention

All organic solvents were degassed and saturated with nitrogen prior to use. 5,10,15,20-Tetra(4-pyridyl)porphyrin (TPP) was purchased from Fluka.

The following starting materials were prepared according to published methods, as indicated.

$[Ru(\eta^6\text{-}C_6H_6)(\mu\text{-}Cl)Cl]_2$: Zelonka, R. A.; Baird, M. C. Can. J. Chem., 1972, 50, 3063-3072.
$[Ru(\eta^6\text{-}C_6H_5CH_3)(\mu\text{-}Cl)Cl]_2$; Bennett, M. A.; Smith, A. K. J. Chem. Soc.; Dalton Trans., 1974, 233-241.
$[Ru(\eta^6\text{-}p\text{-}Pr^iC_6H_4Me)(\mu\text{-}Cl)Cl]_2$: Zelonka, R. A.; Baird, M. C. Can. J. Chem., 1972, 50, 3063-3072.
$[Ru(\eta^6\text{-}C_6Me_6)(\mu\text{-}Cl)Cl]_2$: Bennett, M. A.; Huang, T.-N.; Matheson, T. W.; Smith, A. K. Inorg. Synth.; John Wiley: New York, 1982, 21, p. 74-76.
$[Ru(\eta^6\text{-}1,4\text{-}C_6H_4(COOEt)_2)(\mu\text{-}Cl)Cl]_2$: Therrien, B.; Süss-Fink, G. Inorg. Chim. Acta, 2006, 359, 4350-4354.
$[Os(\eta^6\text{-}p\text{-}Pr^iC_6H_4Me)(\mu\text{-}Cl)Cl]_2$: Arthur, T.; Stephenson, T. A. J. Organomet. Chem., 1981, 208, 369-387.
$[Rh(\eta^5\text{-}Cp^*)(\mu\text{-}Cl)Cl]_2$: White, C.; Oliver, A. J.; Maitlis, P. M. J. Chem. Soc.; Dalton Trans., 1973, 1901-1907.
$[Ir(\eta^5\text{-}Cp^*)(\mu\text{-}Cl)Cl]_2$: White, C.; Oliver, A. J.; Maitlis, P. M. J. Chem. Soc.; Dalton Trans., 1973, 1901-1907.

NMR spectra were recorded on a Varian 200 MHz spectrometer. IR spectra were recorded on a Perkin-Elmer 1720X FT-IR spectrometer (4000-400 $cm^{-1}$). Microanalyses were performed by the Laboratory of Pharmaceutical Chemistry, University of Geneva (Switzerland). Electro-spray mass spectra were obtained in positive-ion mode with an LCQ Finnigan mass spectrometer.

Example 1: $[Ru_4(\eta^6\text{-}C_6H_6)_4(TPP)Cl_8]$ (**1**)

A mixture of $[Ru(\eta^6\text{-}C_6H_6)(\mu\text{-}Cl)Cl]_2$ (100 mg, 0.2 mmol) and 5,10,15,20-(pyrid-4-yl) porphyrin (TPP) (62 mg, 0.1 mmol) was refluxed in dry methanol (20 ml) for 4 h whereby the brownish purple-colored product was separated out. The compound was filtered and washed with diethylether and dried under vacuum. (Yield 90 mg, 56 %). $^1H$ NMR (DMSO-$d_6$, 200 MHz): δ (ppm) = 9.07 (d, 8H, $^3J_{H\text{-}H}$ = 5.88 Hz, $H_\alpha$, pyridine), 8.92 (s, 8H, CH, pyrrole), 8.29 (d, 8H, $H_\beta$, pyridine), 6.52 (s, 24H, $C_6H_6$), -3.05 (s, 2H, NH). Elemental analysis (%) calc. for $C_{64}H_{50}N_8Cl_8Ru_4$: C 47.48, H 3.11, N 6.92; found: C 47.03, H 3.17, N 6.53.

Example 2: $[Ru_4(\eta^6\text{-}C_6H_5CH_3)_4(TPP)Cl_8]$ (**2**)

It was prepared in the same procedure as described above for **1** using $[Ru(\eta^6\text{-}C_6H_5CH_3)(\mu\text{-}Cl)Cl]_2$ (100 mg, 0.19 mmol) and TPP (59 mg, 0.095 mmol). (Yield 110 mg, 70 %). $^1H$ NMR (DMSO-$d_6$, 200 MHz,): δ (ppm) = 9.09 (d, 8H, $^3J_{H\text{-}H}$ = 4.76 Hz, $H_\alpha$, pyridine), 8.93 (s, 8H, CH, pyrrole), 8.31 (d, 8H, $H_\beta$, pyridine), 6.03-5.97 (m, 12H, toluene), 5.72 (d, 8H, $^3J_{H\text{-}H}$ = 5.52 Hz, toluene), 2.52 (s, 12H, $CH_3$, toluene), -3.04 (s, 2H, NH). Elemental analysis (%) calc. for $C_{68}H_{58}N_8Cl_8Ru_4$: C 48.75, H 3.49, N 6.69; found: C 48.80, H 3.70, N 6.83.

Example 3: $[Ru_4(\eta^6\text{-}p\text{-}Pr^iC_6H_4Me)_4(TPP)Cl_8]$ (**3**)

It was prepared in the same procedure as described above for **1** using $[Ru(\eta^6\text{-}p\text{-}Pr^iC_6H_4Me)(\mu\text{-}Cl)Cl]_2$ (100 mg, 0.163 mmol) and TPP (50.5 mg, 0.082 mmol). (Yield 105 mg, 70 %). $^1H$ NMR (DMSO-$d_6$, 200 MHz): δ (ppm) = 9.07 (d, 8H, $^3J_{H\text{-}H}$ = 5.88 Hz, $H_\alpha$, pyridine), 8.91 (s, 8H, CH, pyrrole), 8.29 (d, 8H, $H_\beta$, pyridine), 5.83 (d, 8H, $^3J_{H\text{-}H}$ = 6.6 Hz, CH, cymene), 5.78 (d, 8H, CH, cymene), 2.83 (sep, 4H, CH, cymene), 2.08 (s, 12H, $CH_3$, cymene), 1.21 (d, 24H, $CH_3$, cymene), -3.07 (s, 2H, NH). Elemental analysis (%) calc. for $C_{80}H_{82}N_8Cl_8Ru_4$: C 52.12, H 4.48, N 6.08; found: C 52.16, H 4.51, N 5.96.

Example 4: $[Ru_4(\eta^6\text{-}C_6Me_6)_4(TPP)Cl_8]$ (**4**)

It was prepared in the same procedure as described above for **1** using $[Ru(\eta^6\text{-}C_6Me_6)(\mu\text{-}Cl)Cl]_2$ (100 mg, 0.15

mmol) and TPP (46.3 mg, 0.075 mmol). (Yield 115 mg, 79 %). $^1H$ NMR ($CD_2Cl_2$, 200 MHz): δ (ppm) = 9.25 (d, 8H, $^3J_{H-H}$ = 6.62 Hz, $H_\alpha$, pyridine), 8.89 (s, 8H, CH, pyrrole), 8.21 (d, 8H, $H_\beta$, pyridine), 2.14 (s, 72H, $C_6Me_6$), -3.00 (s, 2H, NH). Elemental analysis (%) calc. for $C_{88}H_{98}N_8Cl_8Ru_4$: C 54.04, H 5.05, N 5.73; found: C 54.67, 4.90, N 5.16.

Example 5: $[Ru_4(\eta^6\text{-}1,4\text{-}C_6H_4(COOEt)_2)_4(TPP)Cl_8]$ (**5**)

It was prepared in the same procedure as described above for **1** using $[Ru(\eta^6\text{-}1,4\text{-}C_6H_4(COOEt)_2)(\mu\text{-}Cl)Cl]_2$ (118 mg, 0.15 mmol) and TPP (46 mg, 0.075 mmol). (Yield 120 mg, 73 %). $^1H$ NMR ($CD_2Cl_2$, 200 MHz,): δ (ppm) = 9.37 (d, 8H, $^3J_{H-H}$ = 5.44 Hz, $H_\alpha$, pyridine), 8.95 (s, 8H, CH, pyrrole), 8.19 (d, 8H, $H_\beta$, pyridine), 5.73 (s, 16H, $C_6H_4$), 4.28-4.17 (q, 8H, $^3J_{H-H}$ = 6.98 Hz, $CH_2$), 1.33-1.26 (t, 12H, $^3J_{H-H}$ = 6.98 Hz, $CH_3$), -3.21 (s, 2H, NH). Elemental analysis (%) calc. for $C_{88}H_{82}N_8Cl_8O_{16}Ru_4$: C 48.14, H 3.76, N 5.10; found: C 48.35, H 3.84, 4.95.

Example 6: $[Os_4(\eta^6\text{-}p\text{-}Pr^iC_6H_4Me)_4(TPP)Cl_8]$ (**6**)

It was prepared in the same procedure as described above for **1** using $[Os(\eta^6\text{-}p\text{-}Pr^iC_6H_4Me)(\mu\text{-}Cl)Cl]_2$ (150 mg, 0.214 mmol) and TPP (66 mg, 0.11 mmol). (Yield 110 mg, 47 %). $^1H$ NMR (DMSO-$d_6$, 200 MHz): δ (ppm) = 9.07 (d, 8H, $^3J_{H-H}$ = 5.88 Hz, $H_\alpha$, pyridine), 8.92 (s, 8H, CH, pyrrole), 8.29 (d, 8H, $H_\beta$, pyridine), 6.09 (d, 8H, $^3J_{H-H}$ = 5.88 Hz, CH, cymene), 6.00 (d, 8H, CH, cymene), 2.77 (sep, 4H, $^3J_{H-H}$ = 6.98 Hz,CH, cymene), 2.13 (s, 12H, $CH_3$, cymene), 1.21 (d, 24H, $CH_3$, cymene), -3.06 (s, 2H, NH). Elemental analysis (%) calc. for $C_{80}H_{82}N_8Cl_8Os_4$: C 43.68, H 3.76, N 5.09; found: C 43.80, H 3.98, N 5.15.

Example 7: $[Rh_4(\eta^5\text{-}Cp^*)_4(TPP)Cl_8]$ (**7**)

It was prepared in the same procedure as described above for **1** using $[Rh(\eta^5\text{-}Cp^*)(\mu\text{-}Cl)Cl]_2$ (100 mg, 0.16 mmol) and TPP (50 mg, 0.08 mmol). (Yield 110 mg, 73 %). $^1H$ NMR (200 MHz, $CD_2Cl_2$): δ (ppm) = 9.42 (d, 8H, $^3J_{H-H}$ = 6.24 Hz, $H_\alpha$, pyridine), 8.87 (s, 8H, CH, pyrrole), 8.28 (d, 8H, $H_\beta$, pyridine), 1.53 (s, 60H, $C_5Me_5$), -3.01 (s, 2H, NH). Elemental analysis (%) calc. for $C_{80}H_{86}N_8Cl_8Rh_4$: C 51.80, H 4.67, N 6.04; found: C 51.61, H 5.00, N 6.24.

Example 8: $[Ir_4(\eta^5\text{-}Cp^*)_4(TPP)Cl_8]$ (**8**)

It was prepared in the same procedure as described above for **1** using $[Ir(\eta^5\text{-}Cp^*)(\mu\text{-}Cl)Cl]_2$ (100 mg, 0.126 mmol) and TPP (39 mg, 0.06 mmol). (Yield 115 mg, 83 %). $^1H$ NMR (DMSO-$d_6$, 200 MHz,): δ (ppm) = 9.07 (d, 8H, $^3J_{HH}$ = 5.50 Hz, $H_\alpha$, pyridine), 8.92 (s, 8H, CH, pyrrole), 8.29 (d, 8H, $H_\beta$, pyridine), 1.63 (s, 60H $C_5Me_5$), -3.06 (s, 2H, NH). Elemental analysis (%) calc. for $C_{80}H_{86}N_8Cl_8Rh_4$: C 43.43, H 3.92, N 5.06; found: C 43.64, H 3.82, N 5.65.

Example 9: UV-Visible absorption spectra

UV-visible absorption spectra of TPP and compounds (**2**)-(**8**) in dichloromethane at 298 K were recorded on an Uvikon 930 spectrophotometer. The result is seen in Figure 1. Table 1 below provides UV-visible absorption data of the compounds TPP-$H_2$, (**2**), (**4**), (**5**), (7).

**Table 1**: UV-visible absorption data of selected compounds in dichloromethane at 298 K

| Compounds | [$\lambda_{max}$ (nm) ($\varepsilon$ x $10^{-3}$ $M^{-1}$ $cm^{-1}$)] | | | | |
|---|---|---|---|---|---|
| | Soret Band | Band IV | Band III | Band II | Band I |
| **Tpp-$H_2$** | 417 (212.9) | 512 (24.2) | 545 (9.4) | 587 (9.8) | 642 (5.7) |
| **2** | 423 (156.5) | 516 (13.6) | 550 (7.7) | 590 (6.7) | 645 (4.4) |
| **4** | 422 (200.0) | 515 (18.7) | 550 (8.0) | 589 (6.2) | 645 (2.7) |
| **5** | 423 (208.8) | 517 (30.3) | 551 (18.6) | 590 (15.8) | 647 (10.3) |
| **7** | 423 (216.2) | 514 (26.7) | 549 (12.1) | 589 (10.6) | 648 (5.7) |

Example 10: X-ray crystallography

Single-crystals of (**4**) and (**7**) were mounted on a Stoe Image Plate Diffraction system equipped with a $\phi$ circle goniometer, using Mo-K$\alpha$ graphite monochromated radiation ($\lambda$ = 0.71073 Å) with $\phi$ range 0-200°, $D_{max}$-$D_{min}$ = 12.45-0.81

Å, increment of 0.8 and 1.0˚, respectively. The structures were solved by direct methods using the program SHELXS-97 (Sheldrick, G. M. Acta Cryst. 1990, A46, 467-473.) The refinement and all further calculations were carried out using SHELXL-97 (G. M. Sheldrick, *SHELXL-97,* University of Göttingen, Göttingen, Germany (1999)). In all cases, the H-atoms were included in calculated positions and treated as riding atoms using the SHELXL default parameters. Examination of the structures with PLATON (Spek, A. L. J. Appl. Cryst. 2003, 36, 7-13) reveals voids between the arene ruthenium porphyrin molecules. Indeed, voids corresponding to solvent molecules were found. Therefore, new data sets corresponding to omission of the missing solvents were generated with the SQUEEZE algorithm (van der Sluis, P.; Spek, A. L. Acta Cryst. 1990, A46, 194-201) and the two structures were refined to convergence. However, in both cases the non-H atoms were refined anisotropically, using weighted full-matrix least-square on $F^2$. Crystallographic details are summarized in Table 2. Figures 2 and 3 of (**4**) and (**7**) are drawn with ORTEP (Farrugia, L. J. J. Appl. Cryst. 1997, 30, 565).

**Table 2**: Crystallographic and selected experimental data for (**4**) and (**7**).

| | **4** | **7** |
|---|---|---|
| Chemical formula | $C_{88}H_{98}Cl_8N_8Ru_4$ | $C_{80}H_{86}Cl_8N_8Rh_4$ |
| Formula weight | 1955.62 | 1854.81 |
| Crystal system | Monoclinic | Triclinic |
| Space group | P $2_1$/n | P -1 |
| Crystal color and shape | purple rod | purple block |
| Crystal size | 0.18 x 0.10 x 0.10 | 0.15 x 0.14 x 0.12 |
| *a* (Å) | 14.890(3) | 12.5575(13) |
| *b* (Å) | 16.0272(12) | 16.237(2) |
| *c* (Å) | 23.640(3) | 16.9186(19) |
| α (˚) | | 64.686(13) |
| β (˚) | 92.25(2) | 73.953(12) |
| γ (˚) | | 71.819(13) |
| *V* ($Å^3$) | 5637.3(15) | 2921.3(6) |
| *Z* | 2 | 1 |
| *T* (K) | 173(2) | 173(2) |
| $D_c$ ($g{\cdot}cm^{-3}$) | 1.152 | 1.054 |
| μ ($mm^{-1}$) | 0.753 | 0.771 |
| Scan range (˚) | $4.16 < 2\theta < 51.86$ | $3.96 < 2\theta < 51.86$ |
| Unique reflections | 10008 | 10590 |
| Reflections used [$I>2\sigma(I)$] | 3384 | 3257 |
| $R_{int}$ | 0.1747 | 0.1659 |
| Finale *R* indices [$I>2\sigma(I)$]* | 0.0896, $wR_2$ 0.2111 | 0.0835, $wR_2$ 0.1955 |
| *R* indices (all data) | 0.1778, $wR_2$ 0.2353 | 0.1788, $wR_2$ 0.2251 |
| Goodness-of-fit | 0.736 | 0.689 |
| Max, Min $\Delta\rho$/e ($Å^{-3}$) | 1.458, -1.014 | 1.027, -1.342 |

* Structures were refined on $F_0^2$: $wR_2 = [\Sigma[w(F_0^2 - F_c^2)^2] / \Sigma w(F_0^2)^2]^{1/2}$, where $w^{-1} = [\Sigma(F_0^2) + (aP)^2 + bP]$ and $P = [\max(F_0^2, 0) + 2F_c^2]/3$

Selected bond lengths (Å) and angles (˚) of compound **4** shown in Figure 2: Ru(1)-Cl(1) 2.416(4), Ru(1)-Cl(2) 2.404(3), Ru(1)-N(1) 2.162(7), Ru(2)-Cl(3) 2.450(4), Ru(2)-Cl(4) 2.395(3), Ru(2)-N(2) 2.100(10); Cl(1)-Ru(1)-Cl(2) 87.67 (13), N(1)-Ru(1)-Cl(1) 87.2(3), N(1)-Ru(1)-Cl(2) 84.3(3), Cl(3)-Ru(2)-Cl(4) 88.47(14), N(2)-Ru(2)-Cl(3) 82.2(3), N(2)-Ru (2)-Cl(4) 85.8(2).

Selected bond lengths (Å) and angles (˚) of compound **7** shown in Figure 3: Rh(1)-Cl(1) 2.405(3), Rh(1)-Cl(2) 2.425(4), Rh(1)-N(1) 2.154(9), Rh(2)-Cl(3) 2.428(4), Rh(2)-Cl(4) 2.431(3), Rh(2)-N(2) 2.103(10); Cl(1)-Rh(1)-Cl(2) 92.30 (13), N(1)-Rh(1)-Cl(1) 88.0(3), N(1)-Rh(1)-Cl(2) 87.0(3), Cl(3)-Rh(2)-Cl(4) 91.72(11), N(2)-Rh(2)-Cl(3) 86.7(3), N(2)-Rh (2)-Cl(4) 89.2(3).

Examples 11: Toxicity of the Present Compounds on Melanoma Cancer Cell Lines

Human ME275 and ME300 melanoma cell lines were from Dr D. Rimoldi, Ludwig Institute of Cancer Research,

Lausanne. All other cell culture reagents were obtained from Gibco-BRL, Basel Switzerland. The cells were routinely grown in RPMI 1640 medium containing 10% foetal calf serum (FCS) and antibiotics. To determine the cytotoxicity of the ruthenium compounds, cells were grown in 48-well cell culture plastic plates (Coming, NY) for 24 h then exposed for 24, 48 or 72 h to the appropriate compound concentration of 1-100 μM. The complexes were dissolved in DMSO as 20 mM stock solution and then diluted in medium containing 5% serum to the required concentration. Experiments were conducted in triplicate. Analysis of cell viability was performed at the end of the incubation time.

Alamar Blue reduction was used to quantify metabolically active cells. Briefly, following treatment, cells were exposed to 10% Alamar Blue (Serotec, Düsseldorf, Germany) added to the cell culture medium without medium change for 120 min, then fluorescence increase was recorded at 37°C in a thermostated multiwell fluorescence reader (Cytofluor, PerSeptive BioSystems) at $\lambda_{ex}/\lambda_{em}$ = 530 nm/580 nm.

Results of non-irradiated cells are shown in Table 3 below. It can be seen that most of the compounds strongly reduce cell viability, even in absence of irradiation.

**Table 3** IC50 values of melanomas after 72 h exposure to organometallic porphyrin complexes **1-7** in the dark.

| Complex | ME300 | ME275 |
|---|---|---|
| **1** | 34.4 ± 2.1 | 102.4 ± 16.7 |
| **2** | 84.9 ± 7.2 | > 100 |
| **3** | > 100 | > 100 |
| **4** | 39.4 ± 5.7 | 29.6 ± 6.2 |
| **5** | 31.2 ± 3.7 | 79.0 ± 12.4 |
| **6** | 20.5 ± 3.2 | 46.1 ± 6.3 |
| **7** | > 100 | > 100 |

The effect of concentration is illustrated at the example of compounds **1** and **4** in Figures 4 and 5. These figures show dose response curves of ME300 and ME275 melanomas after 72 h of exposure to the porphyrin complexes in the dark.

These figures show that toxicity increases with dose, with cell viability being reduced by at least 30% at a concentration of 100 μM of compounds **1** or **4**.

Example 13: Phototoxicity

For determining phototoxicity, cells were grown in multiwell cell culture plastic plates (Coming, NY) for 24 h. The media was replaced with fresh medium containing the ruthenium complexes at 10 μM concentration and the cells were exposed to the complexes for a further 24 h. Thereafter, the media was aspirated, the cells washed once with PBS and replaced by DMEM without phenol red. One set of cells were irradiated at 400-800 nm for 10 min using a 200 mW halogen light source (Richard Wolf 5133 Combilight PDD), equivalent to a fluence level of 1.2 J/cm$^2$, while an identical set was kept in the dark as control. Experiments were conducted in triplicate. Analysis of cell viability was performed after a further incubation of 24 h after irradiation.

The results are shown in Table 4 below.

**Table 4**. Comparison of the viability of treated ME275 and ME300 cells (vs control) which are kept in darkness or exposed to white light irradiation.[a]

| Compounds | ME275 | | ME300 | |
|---|---|---|---|---|
| | **dark** | **irradiated** | **dark** | **irradiated** |
| **1** | 101 ± 5 % | 0 ± 0.2 %[b] | 88 ± 12 % | 0 ± 0.4 %[b] |
| **2** | 95 ± 9 % | 0 ± 0.7 %[b] | 92 ± 5 % | 0 ± 0.6 %[b] |
| **3** | 80 ± 9 % | 21 ± 7 % | 91 ± 4 % | 0 ± 0.2 %[b] |
| **4** | 85 ± 2 % | 21 ± 4 % | 63 ± 8 % | 0 ± 1.1 %[b] |
| **5** | 128 ± 7 % | 0 ± 0.7 %[b] | 88 ± 9 % | 0 ± 0.4 %[b] |
| **6** | 97 ± 22 % | 0 ± 0.2 %[b] | 78 ± 5 % | 0 ± 0.2 %[b] |

(continued)

| Compounds | ME275 | | ME300 | |
|---|---|---|---|---|
| | dark | irradiated | dark | irradiated |
| 7 | 108 ± 31 % | 64 ± 2 % | 93 ± 11 % | 65 ± 8 % |

[a] Irradiation at fluence level of 1.2 J/cm$^2$ white light (400-800 nm).
[b] Alamar blue dye fluorescent levels were below background values and therefore stated as zero to indicate absence of viable cells.

## Claims

**1.** An organometallic compound comprising a structure of formula (I):

$$[M_n\ Mc\ (A)_n\ (L)\ (B)_p\ (C)_q] \qquad (I)$$

which is charged or neutral and which may be present in the form of a salt and/or an optically resolved enantiomer, and, wherein:

M is a transition metal selected from Ru, Rh, Os, Ir, and Fe, preferably Ru;
n is an integer of 1 to 12 if L corresponds to formula (II) below and an integer of 1 to 16 if L corresponds to formula (III) below and designs the number of metal atoms M present in compound (I);
Mc is an optional metal in the centre of L, which may be selected from the same metals as M, and in addition from the metals Zn, Ni, Co, Cu, Ag, Au, Cd, Hg, Pd, Pt.
A and L are ligands of M;
B and C are, independently of each other, optional ligands of M, with p and q, independently of each other, being 0 or an integer of 1 to n.
wherein, if n > 1, all n ligands A and all n metals M in the compound of formula (I) can be the same or different; and, accordingly, wherein, if p and/or q > 1, all p ligands B and/or all q ligands B, respectively, in the compound of formula (I) can be the same or different;
A is an $\eta^6$- arene or a $\eta^5$-cyclopentadienyl, or a $\eta^5$-cyclopentadienyl derivative;
B and C are, independently of each other, freely selectable ligands capable of binding to M,
wherein:

B and C may be covalently connected with each other so as to form a single, bidentate chelating ligand (B-C), or, alternatively,
any one or both of B and C, may be covalently bound to A, so as to form a multidentate chelating ligand (A-C), (A-B) or (A-B-C);

L is a porphyrin derivative of formula (II) or a phtalocyanine derivative of formula (III) below:

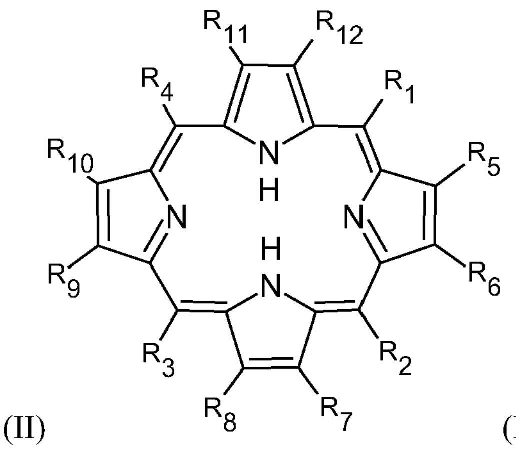

(II)

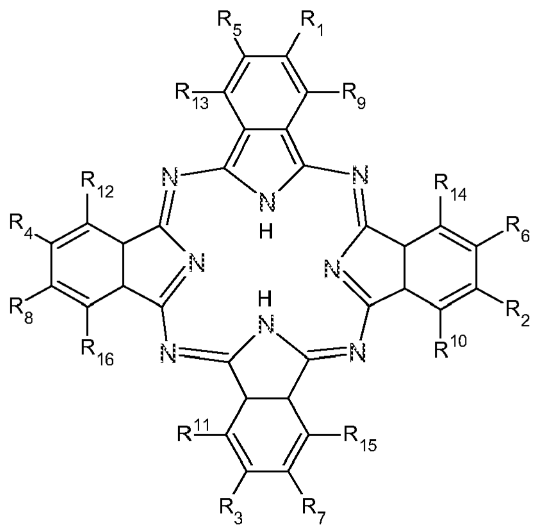

(III)

wherein:

m residues of $R_1$ - $R_{12}$ if L = (II) or of $R_1$ - $R_{16}$ if L = (III) are linkers comprising, all m residues together, at least n donor groups for M, wherein each of said at least n donor groups is selected independently from S-, O-, N- and/or P-donor groups, and,
m is an integer of 1 to 12 if L = (II) and an integer of 1 to 16 if L = (III),
wherein:
two or more (r) of said m residues of $R_1$ - $R_{12}$ if L = (II) or of $R_1$ - $R_{16}$ if L = (III) may, independently of the remaining (m - r) residues, be covalently connected with each other to form a system comprising at least one ring fused to the porphyrin structure of formula (II) or to the phtalocyanine structure of formula (III), respectively;
and, wherein:
(12-m) residues of $R_1$ - $R_{12}$ if L = (II) or (16-m) of $R_1$ - $R_{16}$ if L = (III), respectively, are selected, independently of each other, from H, C1-C20 alkyls, C2-20 alkenyls, C2-C20 alkynyl, C6-C22 aryls, said alkyls, alkenyls, alkynyls and aryls being optionally substituted and optionally comprising one or more heteroatoms, said optinally substituted alkyls, alkenyls, alkynyls being linear or cyclic.

**2.** The organometallic compound of claim 1, wherein A is a compound of formula (IV) or (V):

(IV)

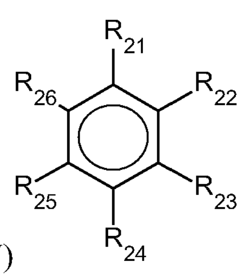

(V)

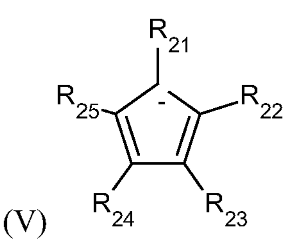

wherein, if A is (IV), any one of $R_{21}$-$R_{26}$, or, if A is (V), respectively, any one of $R_{21}$-$R_{25}$, is independently selected from H, C1-C20 alkyl, C2-20 alkenyl, C2-C20 alkynyl, C6-C22 aryl, said alkyl, alkenyl, alkynyl being linear or cyclic, and said alkyl, alkenyl, alkynyl and aryls being optionally substituted and optionally comprising one or more heteroatoms,
wherein any of the residues $R_{21}$-$R_{26}$ or $R_{21}$-$R_{25}$, respectively may be covalently connected with one or more other residues of $R_{21}$-$R_{26}$ or $R_{21}$-$R_{25}$, respectively, to form one or several rings fused to the ring of formula (IV) or (V), respectively.

**3.** The organometallic compound of any of the preceding claims, wherein:

n is an integer from 1 to 4,
m = n,
L is (II),
all n residues are selected from R1-R4, and,
each of the n linkers comprises at least one S-, O-, N- or P-donor group.

**4.** The organometallic compound of any of the preceding claims, wherein each of said linkers comprises, independently from each other, at least one aromatic N-ring or at least one amine functioning as said N-donor group.

**5.** The organometallic compound of any of the preceding claims, wherein A and B are, independently of each other, selected from H, F, Cl, Br, I, OH, OMe, OEt, OBu, $OH_2$, $CH_3CN$, $(CH_3)_2CO$, DMSO, THF, CO, $CF_3SO_3$, $C_2O_4$, $PMe_3$, $PEt_3$, $PBu_3$, $PPh_3$, $PCy_3$, $HPPh_2$, $HPCy_2$, acetate, acetylacetonate, pyridine, pyrimidine, pyrazine, imidazole, pyridazine, phthlalazine, quinoxaline, 1,5-naphthyridine, 1,8-naphthyridine, 2,2'-bipyrimidine, phenazine, azobenzene, 2,2'-azopyridine, 2,3-di-(2'-pyridino)quinoxaline, diphenylphosphineethane, pyrazinecarboxylic acid, pyrazinedicarboxylic acid, DABCO, ethylenediamine, 1,3,5-triaza-7-phosphatricyclo[3.3.1.1$^{3,7}$]decane.

**6.** The organometallic compound of any of the preceding claims, wherein M is $Ru^{II}$, n = m = 4, R1 to R4 are all identical and each is a linker comprising one S-, O-, N- or P-donor group.

**7.** The organometallic compound of any of the preceding claims, which is [$Ru_4$ $A_4$ (L) $B_4$ $C_4$], with A, B, and C being defined as above.

**8.** The organometallic compound of any of the preceding claims, which is [$Ru_4$ ($\eta^6$-arene)$_4$ (TPP) $Cl_8$], wherein TPP is 5,10,15,20-tetra(4-pyridyl)porphyrin.

**9.** The organometallic compound of any of the preceding claims for use as a pharmaceutically active principle, in particular as a medicament.

**10.** The organometallic compound of any of the preceding claims, for use in the therapeutic treatment of cancer.

# Figure 1

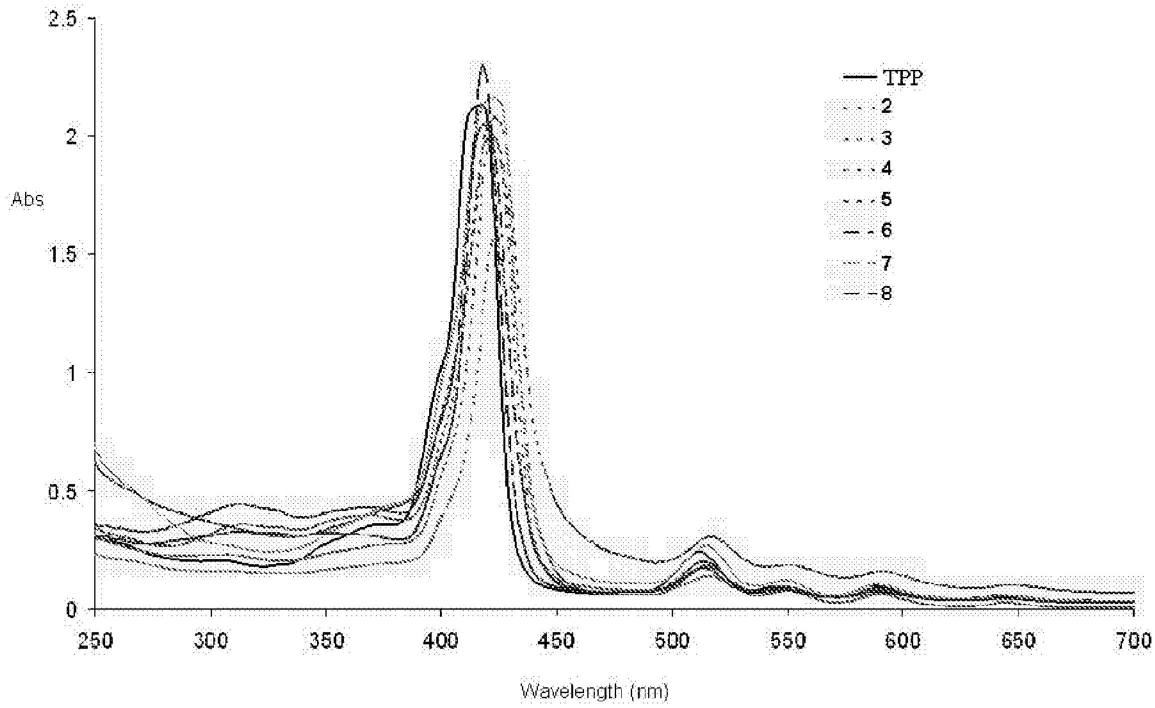
2.5
2
1.5
1
0.5
0
Abs
250
300
350
400
450
500
550
600
650
700
Wavelength (nm)
TPP
2
3
4
5
6
7
8

# Figure 2

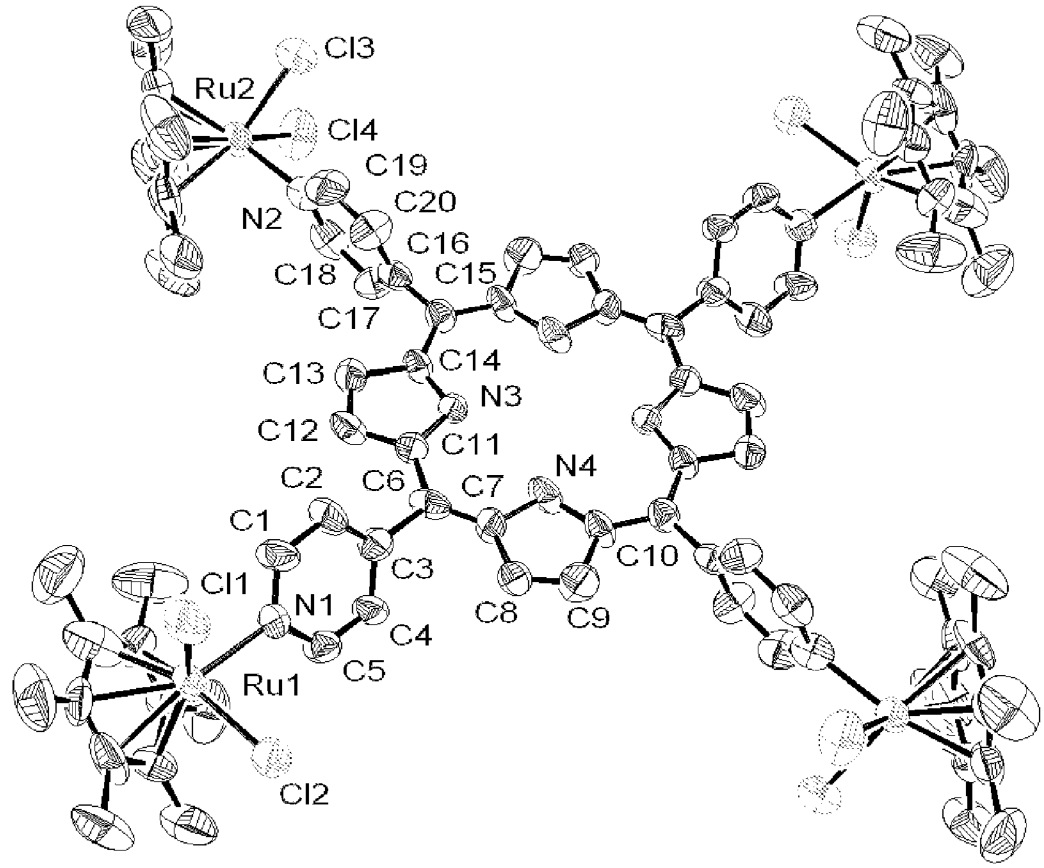

# Figure 3

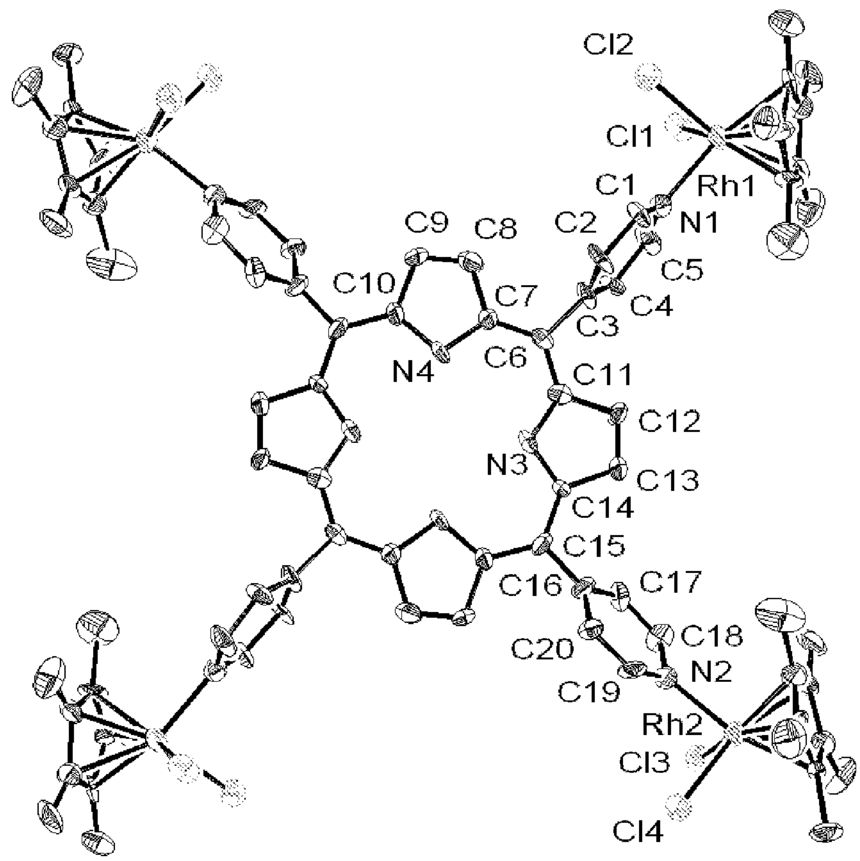

# Figure 4

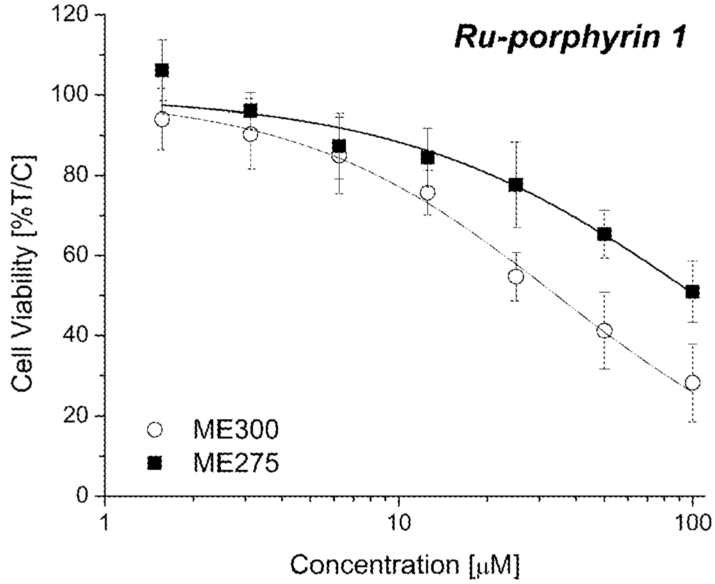


# Figure 5

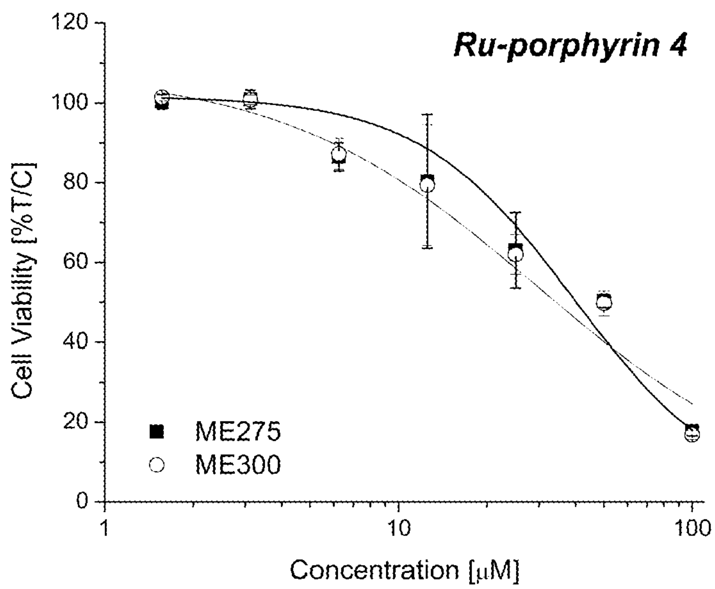

European Patent Office

# EUROPEAN SEARCH REPORT

Application Number
EP 07 10 1258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | US 7 087 214 B2 (BART KARL-CHRISTIAN [DE] ET AL BART KARL CHRISTIAN [DE] ET AL) 8 August 2006 (2006-08-08) * the whole document * ----- | 1 | INV. C07F17/02 C07F15/00 A61K31/28 A61P35/00 |
| D,A | WO 02/40494 A (UNIV YORK [GB]; DYSON PAUL [GB]) 23 May 2002 (2002-05-23) * the whole document * ----- | 1 | |
| | | | TECHNICAL FIELDS SEARCHED (IPC) |
| | | | C07F A61K A61P |

The present search report has been drawn up for all claims

1

| Place of search | Date of completion of the search | Examiner |
|---|---|---|
| The Hague | 15 June 2007 | Rinkel, Bert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 10 1258

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2007

| Patent document cited in search report | Publication date | Patent family member(s) | Publication date |
|---|---|---|---|
| US 7087214 B2 | 08-08-2006 | US 2004023942 A1 | 05-02-2004 |
| WO 0240494 A | 23-05-2002 | AU 1511102 A | 27-05-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7087214 B
- WO 0240494 A
- US 20040023942 A
- WO 2006018649 A


### Non-patent literature cited in the description


- **JANG-QIANG WANG et al.** Porphyrin-carborane organometallic assemblies based on 1,2-dicarba-closo-dodecacarborane (12) ligands. *Chem. Comm.,* 2006, 162-164
- **ZELONKA, R. A. ; BAIRD, M. C.** *Can. J. Chem,* 1972, vol. 50, 3063-3072
- **BENNETT, M. A. ; SMITH, A. K.** *J. Chem. Soc.; Dalton Trans.,* 1974, 233-241
- **BENNETT, M. A. ; HUANG, T.-N. ; MATHESON, T. W. ; SMITH, A. K.** Inorg. Synth. John Wiley, 1982, vol. 21, 74-76
- **THERRIEN, B. ; SÜSS-FINK, G.** *Inorg. Chim. Acta,* 2006, vol. 359, 4350-4354
- **ARTHUR, T. ; STEPHENSON, T. A.** *J. Organomet. Chem.,* 1981, vol. 208, 369-387
- **WHITE, C. ; OLIVER, A. J. ; MAITLIS, P. M.** *J. Chem. Soc.; Dalton Trans.,* 1973, 1901-1907
- **SHELDRICK, G. M.** *Acta Cryst.,* 1990, vol. A46, 467-473
- **SPEK, A. L.** *J. Appl. Cryst.,* 2003, vol. 36, 7-13
- **VAN DER SLUIS, P. ; SPEK, A. L.** *Acta Cryst.,* 1990, vol. A46, 194-201
- **FARRUGIA, L. J.** *J. Appl. Cryst,* 1997, vol. 30, 565